(19) Europäisches Patentamt
European Patent Office
Office européen des brevets

(11) EP 3 322 764 B1

(12) EUROPÄISCHE PATENTSCHRIFT

(45) Veröffentlichungstag und Bekanntmachung des Hinweises auf die Patenterteilung:
**09.09.2020 Patentblatt 2020/37**

(21) Anmeldenummer: **16739103.6**

(22) Anmeldetag: **14.07.2016**

(51) Int Cl.:
***C09J 133/14*** *(2006.01)* ***C08F 220/18*** *(2006.01)*

(86) Internationale Anmeldenummer:
**PCT/EP2016/066707**

(87) Internationale Veröffentlichungsnummer:
**WO 2017/009401 (19.01.2017 Gazette 2017/03)**

# (54) SCHMELZKLEBSTOFF, ENTHALTEND POLY(METH)ACRYLAT AUS ALKY(METH)ACRYLATEN UND AUS BESTIMMTEN HETEROCYCLISCHE GRUPPEN ENTHALTENDEN (METH)ACRYLATEN

HOT-MELT ADHESIVE, COMPRISING A POLY(METH)ACRYLATE HAVING ACRYLIC UNITS CONTAINING HETEROCYCLIC GROUPS

ADHÉSIF FUSIBLE CONTENANT UN POLY(MÉTH)ACRYLATE CONSTITUÉ PAR DES (MÉTH)ACRYLATES D'ALKYLE ET DES (MÉTH)ACRYLATES CONTENANT DES GROUPES HÉTÉROCYCLIQUES DÉTERMINÉS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **16.07.2015 EP 15177088**

(43) Veröffentlichungstag der Anmeldung:
**23.05.2018 Patentblatt 2018/21**

(73) Patentinhaber: **BASF SE**
**67056 Ludwigshafen am Rhein (DE)**

(72) Erfinder:
- **LICHT, Ulrike**
  **68309 Mannheim (DE)**
- **WULFF, Dirk**
  **67105 Schifferstadt (DE)**
- **MISSKE, Andrea**
  **67346 Speyer (DE)**

(74) Vertreter: **BASF IP Association**
**BASF SE**
**G-FLP-C006**
**67056 Ludwigshafen (DE)**

(56) Entgegenhaltungen:
**EP-A2- 1 132 444**
**DE-A1- 10 339 137**
**US-A1- 2003 153 642**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 322 764 B1

## Beschreibung

Die Erfindung betrifft einen Haftklebstoff, insbesondere in Form eines Schmelzklebstoffs, enthaltend mindestens ein Poly(meth)acrylat, welches gebildet ist aus C1-bis C18-Alkyl-(meth)-acrylaten, und bestimmten heterocyclische Gruppen enthaltenden (Meth)acrylaten. Die Erfindung betrifft auch ein entsprechendes Poly(meth)acrylat mit einpolymerisiertem Fotoinitiator sowie die Verwendung zur Herstellung von Klebstoffen, vorzugsweise von Haftklebstoffen zur Herstellung von Haftklebstoffprodukten.

Bei Acrylat-Haftklebstoffen ist ein ausgewogenes Verhältnis von Adhäsionsverhalten (Haftung zum Substrat) zu Kohäsionsverhalten (innere Festigkeit in der Klebstoffschicht) gewünscht. So soll z.B. eine gute Adhäsion zu Stahloberflächen vorliegen, ein gutes Auffließen auf die Substratoberfläche gewährleistet sein und gleichzeitig ein hohe Kohäsion bei Raumtemperatur und bei erhöhten Temperaturen, z.B. bei 70°C vorliegen. Für eine gute Auftragbarkeit soll gleichzeitig als Randbedingung eine möglichst niedrige Schmelzviskosität (im Falle von Schmelzklebstoffen, z.B. sogenannten 100%igen UV-hotmelts) bzw. eine möglichst niedrige Dispersionsviskosität bei hohem Feststoffgehalt (in Falle von wässrigen Haftklebstoffdispersionen) eingehalten werden.

Strahlungsvernetzbare Schmelzklebstoffe auf der Basis von (Meth)acrylatpolymeren und deren Verwendung als Haftklebstoffe sind beispielsweise bekannt aus DE 102004058070, EP-A 246 848, EP-A 377 191, EP-A 445 641 , EP 1132444 A2 oder WO 01/23488. Wässrige Haftklebstoffdispersionen und Polymerdispersionen zum Kaltsiegeln mit einem Gehalt an Acrylatpolymeren aus u.a. bestimmte heterocyclische Substituenten enthaltenden (Meth)acrylatmonomeren sind bekannt aus WO 2012/139941, WO 2012/140174 und WO 2014/154507.

Der Erfindung lag die Aufgabe zugrunde, Polymere für Haftklebstoffe zur Verfügung zu stellen, mit einer verbesserten Kohäsionswirkung bei gleichzeitig guter Adhäsion, gutem Auffließverhalten und möglichst niedriger Auftrageviskosität.

Die Aufgabe wird erfindungsgemäß gelöst durch einen Schmelzklebstoff, enthaltend mindestens ein Poly(meth)acrylat, welches gebildet ist aus

(a) mindestens einem Monomer M1, ausgewählt aus C1- bis C18-Alkyl-(meth)acrylaten, und
(b) mindestens einem Monomer M2 der allgemeinen Formel

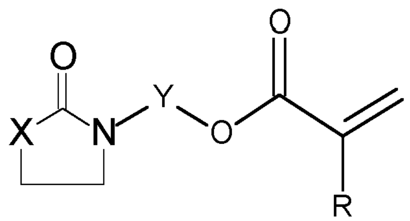

wobei X für O oder $CH_2$ steht,
Y für eine divalente organische Verbindungsgruppe, vorzugsweise eine substituierte oder nicht substituierte, lineare oder verzweigte Kette mit 2 bis 12 C-Atomen steht, die durch O, N, oder S-Atome unterbrochen sein kann, und
R für H oder Methyl steht,

und optional weiteren, von den Monomeren M1 und M2 verschiedenen Monomeren.

Gegenstand der Erfindung ist auch ein Poly(meth)acηlat, geeignet für eine Verwendung als oder in einem Schmelzklebstoff, wobei das Poly(meth)acrylat, gebildet ist aus

(a) mindestens einem Monomer M1, ausgewählt aus C1- bis C18-Alkyl-(meth)acrylaten, und
(b) mindestens einem Monomer M2 der allgemeinen Formel

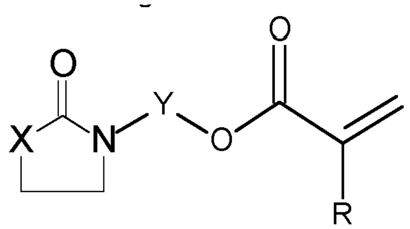

wobei X für O oder $CH_2$ steht,
Y für eine divalente organische Verbindungsgruppe, vorzugsweise eine substituierte oder nicht substituierte, lineare oder verzweigte Kette mit 2 bis 12 C-Atomen steht, die durch O, N, oder S-Atome unterbrochen sein kann, und
R für H oder Methyl steht,

und mindestens einem in das Poly(meth)acrylat einpolymerisierten Fotoinitiator.

Vorzugsweise ist das Poly(meth)acrylat gebildet aus

(a) zu mindestens 50 Gew.% bezogen auf die Summe aller Monomere, aus den Monomeren M1, und
(b) zu mindestens 0,1 Gew.% bezogen auf die Summe aller Monomere, aus den Monomeren M2.

Das Poly(meth)acrylat weist vorzugsweise eine Glasübergangstemperatur von kleiner oder gleich 10 °C, insbesondere von -60 bis +10 °C auf.

Die Glasübergangstemperatur wird durch Differential Scanning Calorimetrie (ASTM D 3418-08, sogenannte "midpoint temperature") bestimmt. Die Glasübergangstemperatur des Polymers ist die bei Auswertung der zweiten Heizkurve (Heizrate 20° C/min) erhaltene Glasübergangstemperatur. Im Falle von (strahlungs)vernetzbaren Polymeren bezieht sich die Glasübergangstemperatur auf diejenige des unvernetzten Polymers.

Der Klebstoff ist vorzugsweise ein Haftklebstoff. Ein Haftklebstoff ist ein viskoelastischer Klebstoff, dessen abgebundener Film bei Raumtemperatur (20°C) in trockenem Zustand permanent klebrig und klebfähig bleibt. Die Klebung auf Substraten erfolgt sofort durch leichten Anpressdruck.

Der Schmelzklebstoff kann strahlungsvernetzbar, vorzugsweise UV-vernetzbar sein. Der Begriff strahlungsvernetzbar bedeutet, dass der Schmelzklebstoff mindestens eine Verbindung mit mindestens einer strahlungsempfindlichen Gruppe enthält und bei Bestrahlung eine Vernetzungsreaktion induziert wird. Die Bestrahlung zur Vernetzung erfolgt vorzugsweise mit aktinischer Strahlung, vorzugsweise UV-Licht, insbesondere UV-C Strahlung. Ein strahlungsvernetzbarer Schmelzklebstoff enthält vorzugsweise mindestens einen Fotoinitiator. Der Fotoinitiator kann als nicht an das Poly(meth)acrylat gebundenes Additiv vorliegen und/oder der Fotoinitiator kann in das Poly(meth)acrylat einpolymerisiert sein.

Schmelzklebstoffe, die auch als Heißklebstoffe, Heißkleber, Heißleim oder Hotmelt bekannt sind, sind lösungsmittelfreie (d.h. nicht in Wasser oder organischen Lösungsmitteln gelöst oder dispergiert) und bei Raumtemperatur mehr oder weniger feste Produkte, die im heißen Zustand hinreichend fluide sind und aufgrund der damit verbundenen Viskositätserniedrigung auf eine Klebefläche aufgetragen werden können, und beim Abkühlen die Klebeverbindung herstellen, wobei strahlungsvernetzbare Schmelzklebstoffe dabei noch bestrahlt werden können.

Im Folgenden werden gelegentlich die Bezeichnung "(Meth)acryl..." und ähnliche Bezeichnungen als abkürzende Schreibweise verwendet für "Acryl... oder Methacryl... ". In der Bezeichnung Cx-Alkyl(meth)acrylat und analogen Bezeichnungen bedeutet x die Anzahl der C-Atome der Alkylgruppe.

Mengenangaben zu Monomeren eines Polymers beziehen sich, soweit nicht explizit etwas anderes angegeben ist, auf 100 Gewichtsteile der Summe aller Monomere.

Das (vorzugsweise strahlungsvernetzbare) Poly(meth)acrylat ist zu mindestens 50 Gew.% oder zu mindestens 60 Gew.%, oder zu mindestens 80 Gew.% aus C1- bis C18-Alkyl(meth)acrylaten (Monomere M1) gebildet. Bevorzugt sind C1- bis C10-Alkyl(meth)acrylate, oder C4- bis C10-Alkyl(meth)acrylate, oder C1-C8 Alkyl(meth)acrylate, insbesondere C4- bis C8-Alkyl(meth)-acrylate, z.B. Methyl(meth)acrylat, Ethylacrylat, n-Butylacrylat, n-Hexylacrylat, 2-Propylhexylacrylat und 2-Ethylhexylacrylat und deren Mischungen. Besonders bevorzugt sind n-Butylacrylat und 2-Ethylhexylacrylat.

In einer Ausführungsform der Erfindung besteht das Poly(meth)acrylatpolymer zu mindestens 80 Gew.% aus mindestens einem Acrylat welches ausgewählt ist aus der Gruppe bestehend aus n-Butylacrylat, n-Hexylacrylat, 2-Ethylhexylacrylat, 2-Propylhexylacrylat und deren Mischungen oder das Poly(meth)acrylatpolymer besteht zu mindestens 90 Gew.% aus 2-Ethylhexylacrylat oder n-Butylacrylat.

Das (vorzugsweise strahlungsvernetzbare) Poly(meth)acrylat ist vorzugsweise zu mindestens 0,1 Gew.%, zu mindestens 0,2 Gew.%, zu mindestens 1 Gew.% oder zu mindestens 5 Gew.% und vorzugsweise bis zu 30 Gew.% oder bis zu 25 Gew.% aus den Monomeren M2 aufgebaut.

Im Falle von Schmelzklebstoffen ist das Poly(meth)acrylat vorzugsweise zu 1 bis 30 Gew.% oder von 5 bis 25 Gew.% aus den Monomeren M2 aufgebaut. Im Falle von wässrigen Dispersionsklebstoffen ist das Poly(meth)acrylat vorzugsweise zu 0,1 bis 5 Gew.% oder von 0,2 bis 2 Gew.% aus den Monomeren M2 aufgebaut.

Das Monomer M2 hat die allgemeine Formel

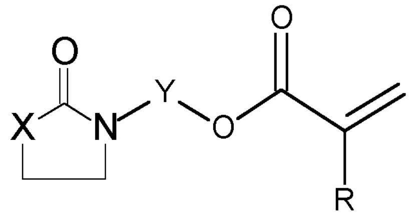

wobei X für O oder $CH_2$ steht,
Y für eine divalente organische Verbindungsgruppe, vorzugsweise eine substituierte oder nicht substituierte, lineare oder verzweigte Kette mit 2 bis 12 C-Atomen steht, die durch O, N, oder S-Atome unterbrochen sein kann, und
R für H oder Methyl steht.

Vorzugsweise ist Y eine lineare oder verzweigte Alkylengruppe mit 2 bis 12 C-Atomen oder mit 2 bis 6 C-Atomen, z.B. eine lineare Alkylengruppe mit 2 bis 6 C-Atomen, z.B. Ethylen, Propylen, Butylen, Pentylen oder Hexylen. Besonders bevorzugt sind Monomere der folgenden Formeln:

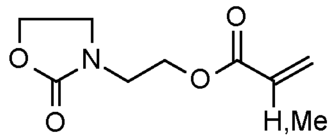

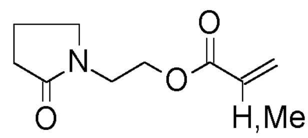

Besonders bevorzugt sind 2-(2-Oxooxazolidin-3-yl)ethylacrylat, 2-(2-Oxopyrrolidin-1-yl)ethylacrylat, 2-(2-Oxooxazolidin-3-yl)ethylmethacrylat und 2-(2-Oxopyrrolidin-1-yl)ethylmethacrylat oder Mischungen dieser Monomere.
Das Poly(meth)acrylatpolymer kann aus weiteren, ethylenisch ungesättigten Verbindungen als Aufbaukomponenten gebildet sein, z.B. Vinylester von bis zu 20 C-Atome enthaltenden Carbonsäuren, Vinylaromaten mit bis zu 20 C-Atome, ethylenisch ungesättigten Nitrile, Vinylhalogenide, Vinylether von 1 bis 10 C-Atome enthaltenden Alkoholen, aliphatischen Kohlenwasserstoffe mit 2 bis 8 C-Atomen und 1 oder 2 Doppelbindungen oder Mischungen dieser Monomeren. Vinylester von Carbonsäuren mit 1 bis 20 C-Atomen sind z. B. Vinyllaurat, Vinylstearat, Vinylpropionat, Versaticsäurevinylester und Vinylacetat. Als vinylaromatische Verbindungen kommen z.B. Vinyltoluol, $\alpha$-und p-Methylstyrol, alpha-Butylstyrol, 4-n-Butylstyrol, 4-n-Decylstyrol und vorzugsweise Styrol in Betracht. Beispiele für Nitrile sind Acrylnitril und Methacrylnitril. Die Vinylhalogenide sind mit Chlor, Fluor oder Brom substituierte ethylenisch ungesättigte Verbindungen, bevorzugt Vinylchlorid und Vinylidenchlorid. Als Vinylether zu nennen sind z.B. Vinylmethylether oder Vinylisobutylether. Bevorzugt sind Vinylether von 1 bis 4 C-Atome enthaltenden Alkoholen. Als Kohlenwasserstoffe mit 2 bis 8 C-Atomen und zwei olefinischen Doppelbindungen seien Butadien, Isopren und Chloropren genannt.
Das Poly(meth)acrylatpolymer kann zusätzlich zu den Monomeren M1 und M2 vorzugsweise aus mindestens einem Monomeren mit polaren Gruppen gebildet sein. Monomere mit polaren Gruppen sind z.B. Monomere, bei denen die polaren Gruppen ausgewählt sind aus Carbonsäuregruppen, Carbonsäureanhydridgruppen, Hydroxygruppen, Amidgruppen, Urethangruppen, Harnstoffgruppen, Piperidinylgruppen, Piperazinylgruppen, Morpholinylgruppen, Imidazolylgruppen und Kombinationen von zwei oder mehr der genannten Gruppen.
Die Monomeren mit polaren Gruppen haben vorzugsweise eine Wasserlöslichkeit bei 21 °C größer 5 g/Liter oder größer 10 g/Liter. Das Poly(meth)acrylatpolymer ist vorzugsweise zu 0,1 bis 30 Gew.%, besonders bevorzugt zu 0,3 bis 25 Gew.% oder zu 0,5 bis 15 Gew.% oder von größer oder gleich 1 bis 15 Gew.% aus den Monomeren mit polaren Gruppen gebildet.
Als weitere Monomere in Betracht kommen insbesondere Monomere mit Carbonsäure-, Sulfonsäure- oder Phosphonsäuregruppen. Bevorzugt sind Carbonsäuregruppen. Genannt seien z. B. Acrylsäure, Methacrylsäure, Itaconsäure, Maleinsäure oder Fumarsäure. Bevorzugte Monomere mit Carbonsäuregruppen sind Acrylsäure und Methacrylsäure.
Weitere Monomere sind z. B. auch (Meth)acrylamid und Hydroxylgruppen enthaltende Monomere, insbesondere C1-C10-Hydroxyalkyl-(meth)acrylate. Bevorzugte Monomere mit Hydroxygruppen sind C1-C10-Hydroxyalkyl(meth)acrylate, insbesondere Hydroxyethyl(meth) acrylat und Hydroxypropyl(meth) acrylat. Darüber hinaus seien Phenyloxyethylglykolmono-(meth)-acrylat, Glycidylacrylat, Glycidylmethacrylat, Amino-(meth)acrylate wie 2-Aminoethyl(meth)-acrylat genannt. Monomere, die außer der Doppelbindung noch weitere funktionelle Gruppen tragen, z. B. Isocyanat-, Amino-, Hydroxy-, Amid- oder Glycidyl-, können z.B. die Haftung auf Substraten verbessern.
In einer Ausführungsform ist der Schmelzklebstoff bzw. das Poly(meth)acrylat strahlungsvernetzbar, z.B. durch Bestrahlung mit UV-Licht. Der Schmelzklebstoff enthält dann mindestens einen Fotoinitiator. Der Fotoinitiator kann dabei ausschließlich als nicht an das Poly(meth)-acrylat gebundenes Additiv vorliegen. In einer anderen Ausführungsform

liegt der Fotoinitiator ausschließlich als in das Poly(meth)acrylat einpolymerisierte Komponente vor. Es ist aber auch eine Kombination dieser beiden Ausführungsformen möglich. Strahlungsvernetzbare Poly-(meth)acrylate weisen vor Vernetzung eine Glasübergangstemperatur von vorzugsweise kleiner oder gleich 10 °C, z.B. von -60 bis +10 °C auf.

Zur Strahlungsvernetzung enthält der Schmelzklebstoff einen Fotoinitiator. Der Fotoinitiator ist vorzugsweise in dem Poly(meth)acrylat copolymerisiert. Er kann aber auch ungebunden und lediglich mit dem Polymerisat vermischt sein. Übliche Fotoinitiatoren, die dem Polymerisat als Additiv zugesetzt werden können, sind z.B. Acetophenon, Benzoinether, Benzildialkylketale oder deren Derivate. Der Gehalt des zugemischten Fototinitiators beträgt vorzugsweise 0,05 bis 10 Gew. Teile, besonders bevorzugt 0,1 bis 2 Gew. Teile pro 100 Gew. Teile Poly(meth)acrylat.

Durch Bestrahlung mit energiereichem Licht, insbesondere UV- Licht, bewirkt der Fotoinitiator bzw. die Fotoinitiatorgruppe eine Vernetzung des Polymeren und/oder des Oligomeren, vorzugsweise durch eine chemische Pfropfreaktion der Fotoinitiatorgruppe mit einer räumlich benachbarten Polymer- oder Oligomerkette. Insbesondere kann die Vernetzung durch Einschub einer Carbonylgruppe des Fotoinitiators in eine benachbarte C-H-Bindung unter Ausbildung einer -C-C-O-H Gruppierung erfolgen. Der Wellenlängenbereich, in dem die Fotoinitiatorgruppe aktiviert werden kann, d. h. in dem die Hauptabsorptionsbande der Fotoinitiatorgruppe liegt, ist vorzugsweise 200 bis 450 nm, besonders bevorzugt 250 bis 350 nm, ganz besonders bevorzugt 250 bis 280 nm.

Der Schmelzklebstoff enthält vorzugsweise 0,0001 bis 0,1 mol, besonders bevorzugt 0,0002 bis 0,1, ganz besonders bevorzugt 0,0003 bis 0,01 mol des Fotoinitiators, bzw. der als Fotoinitiator wirksamen, an das Polymer gebundenen Molekülgruppe, pro 100 g Schmelzklebstoff.

Bei dem strahlungsvernetzbaren Poly(meth)acrylat kann es sich um einen Klebstoff auf Basis eines Polymers mit einpolymerisiertem Fotoinitiator handeln. Das Poly(meth)acrylat kann hergestellt werden durch radikalische Polymerisation von ethylenisch ungesättigten Monomeren unter Copolymerisation von mindestens einer strahlungsempfindlichen, radikalisch polymerisierbaren organischen Verbindung. Strahlungsempfindliche, radikalisch polymerisierbare organische Verbindungen werden im Folgenden kurz als polymerisierbarer Fotoinitiator bezeichnet. Der polymerisierbare Fotoinitiator kann durch radikalische Copolymerisation in die Polymerkette von Copolymeren eingebaut werden. Polymerisierbare Fotoinitiatoren haben vorzugsweise folgenden prinzipiellen Aufbau:

A-X-B

wobei A ein einwertiger organischer Rest ist, welcher als strahlungsempfindliche Gruppe vorzugsweise eine Phenongruppe aufweist,
X eine Estergruppe ist, ausgewählt aus -O-C(=O)-, -(C=O)-O- und -O-(C=O)-O-, und
B ein einwertiger organischer Rest ist, welcher eine ethylenisch ungesättigte, radikalisch polymerisierbare Gruppe enthält. Bevorzugte Reste A sind Reste, welche mindestens ein Strukturelement enthalten, welches abgeleitet ist von Phenonen, insbesondere von Acetophenonen oder Benzophenonen. Bevorzugte Reste B enthalten mindestens eine, vorzugsweise genau eine Acryl- oder Methacrylgruppe.

Die ethylenisch ungesättigte Gruppe kann direkt an die Gruppe X gebunden sein. Ebenso kann die strahlungsempfindliche Gruppe direkt an die Gruppe X gebunden sein. Es können sich aber auch zwischen ethylenisch ungesättigter Gruppe und der Gruppe X bzw. zwischen strahlungsempfindlicher Gruppe und Gruppe X jeweils eine Spacergruppe (Abstandshalter) befinden. Die Spacergruppe kann z.B. ein Molekulargewicht von bis zu 500, insbesondere bis zu 300 oder 200 g/Mol aufweisen.

Geeignete Fotoinitiatoren sind z.B. Verbindungen mit Acetophenon- oder Benzophenonstruktureinheiten, beispielsweise beschrieben in EP 377191 A oder EP 1213306 A. Eine bevorzugte Gruppe X ist die Carbonatgruppe -O-(C=O)-O-. Bevorzugte polymerisierbare Fotoinitiatoren sind Verbindungen der Formel F-1:

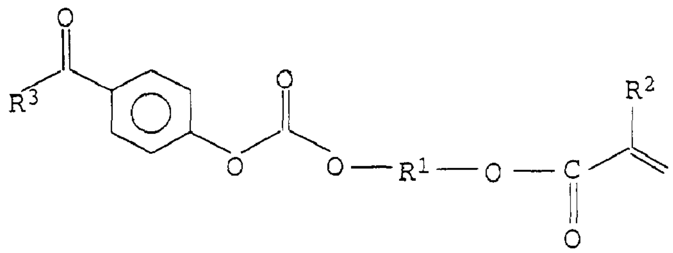

F-1

worin R1 für einen organischen Rest mit bis zu 30 C-Atomen, R2 für ein H-Atom oder eine Methylgruppe und R3 für eine substituierte oder unsubstituierte Phenylgruppe oder für eine C1-C4-Alkylgruppe steht. R1 steht besonders bevorzugt für eine Alkylengruppe, insbesondere für eine C2-C8-Alkylengruppe. R3 steht besonders bevorzugt für eine Methylgruppe oder für eine Phenylgruppe, ganz besonders bevorzugt für eine Phenylgruppe.

Weitere, als copolymerisierbare Fotoinitiatoren geeignete Acetophenon- und Benzophenonderivate sind z.B. solche der Formel F-2

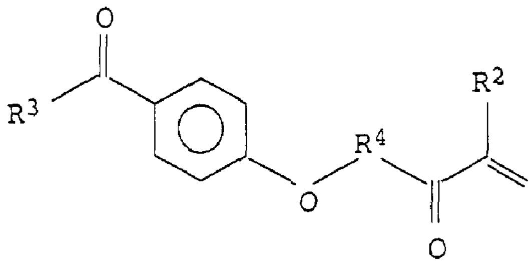

F-2

worin R2 und R3 die obige Bedeutung haben kann und R4 für eine Einfachbindung oder für (-CH2-CH2-O)n stehen kann, wobei n für eine ganze Zahl von 1 bis 12 steht.

Das Poly(meth)acrylat ist im Fall des einpolymerisierten Fotoinitiators vorzugsweise zu 0,05 bis 10 Gew.-% oder zu 0,05 bis 5 Gew.-%, besonders bevorzugt zu 0,1 bis 2 Gew.-% oder zu 0,1 bis 1 Gew.-% aus mindestens einer ethylenisch ungesättigten, copolymerisierbaren Verbindung mit einer Fotoinitiatorgruppe gebildet.

Ein erfindungsgemäß bevorzugtes Poly(meth)acrylat ist gebildet aus

(a1) mindestens 60 Gew.% mindestens eines Monomer M1, welches ausgewählt ist aus der Gruppe bestehend aus n-Butylacrylat, n-Hexylacrylat, 2-Ethyl-hexylacrylat, Propylheptylacrylat und deren Mischungen und
(a2) 0,5 bis 15 Gew.% Monomeren mit polaren Gruppen, wobei die polaren Gruppen ausgewählt sind aus Carbonsäuregruppen,
(b) 0,2 bis 25 Gew.% Monomeren M2, ausgewählt aus 2-(2-Oxooxazolidin-3-yl)ethyl(meth)-acrylat, 2-(2-Oxopyrrolidin-1-yl)ethyl(meth)acrylat und 2-(2-Oxoimidazolidin-1-yl)ethylmethacrylat und Mischungen dieser Monomere und
(c) 0,05 bis 5 Gew.% mindestens eines ethylenisch ungesättigten, copolymerisierbaren Fotoinitiators.

Die Glasübergangstemperatur (Tg) des Polymers liegt bei kleiner oder gleich +10 °C, vorzugsweise im Bereich von -60 bis +10 °C, insbesondere im Bereich von -60 bis 0°C, oder von -55°C bis -10 °C, besonders bevorzugt von -55 °C bis -20 °C. Im Falle von strahlungsvernetzbaren Polymeren bezieht sich die Glasübergangstemperatur auf die unvernetzten Polymere. Die Glasübergangstemperatur lässt sich durch Differential Scanning Calorimetrie bestimmen. Durch die sogenannte Foxgleichung ist es dem Fachmann möglich, Copolymere im geeigneten Tg-Bereich vorab zu identifizieren und diese durch geeignete Variation von Art und Menge der Monomeren gezielt herzustellen. Nach Fox (T.G. Fox, Bull. Am. Phys. Soc. 1956 [Ser. II] 1, Seite 123 und gemäß Ullmann's Encyclopädie der technischen Chemie, Bd. 19, Seite 18, 4. Auflage, Verlag Chemie, Weinheim, 1980) gilt für die Glasübergangstemperatur von höchstens schwach vernetzten Mischpolymerisaten in guter Näherung:

$$1/T_g = x^1/T_g^1 + x^2/T_g^2 + \dots\, x^n/T_g^n,$$

wobei $x^1$, $x^2$, .... $x^n$ die Massenbrüche der Monomeren 1, 2, .... n und $T_g^1$, $T_g^2$, .... $T_g^n$ die Glasübergangstemperaturen der jeweils nur aus einem der Monomeren 1, 2, .... n aufgebauten Polymerisaten in Grad Kelvin bedeuten. Die $T_g$-Werte für die Homopolymerisate der meisten Monomeren sind bekannt und z.B. in Ullmann's Ecyclopedia of Industrial Chemistry, 5. Aufl., Vol. A21, Seite 169, VCH Weinheim, 1992, aufgeführt; weitere Quellen für Glasübergangstemperaturen von Homopolymerisaten bilden z.B. J. Brandrup, E.H. Immergut, Polymer Handbook, 1st Ed., J. Wiley, New York 1966, 2nd Ed. J. Wiley, New York 1975, und 3rd Ed. J. Wiley, New York 1989.

Das Polymer hat vorzugsweise einen K-Wert von größer oder gleich 20, z.B. von 30 bis 80, besonders bevorzugt von 40 bis 60, gemessen in Tetrahydrofuran (1%ige Lösung, 21 °C). Der K-Wert nach Fikentscher ist ein Maß für das Molekulargewicht und die Viskosität des Polymerisats. Dabei erfolgt die Viskositätsmessung mittels eines Kapillarviskosimeters. Durchführungsvorschriften finden sich in DIN EN ISO 1628-1:2012-10.

In einer Ausführungsform enthält der Schmelzklebstoff zusätzlich mindestens ein Oligo(meth)-acrylat, welches eine oder mehrere nicht-acrylische, olefinische C-C-Doppelbindungen aufweist.

Zur Erreichung einer guten viskositätssenkenden Wirkung bei gleichzeitig guter Kohäsion liegt das Gewichtsverhältnis von Poly(meth)acrylat zu Oligo(meth)acrylat vorzugsweise im Bereich von 99:1 bis 50:50, besonders bevorzugt von 95:5 bis 75: 25. Die Oligo(meth)acrylate weisen eine oder mehrere nicht-acrylische, olefinische C-C-Doppelbindungen auf. Sie haben einen K-Wert von kleiner oder gleich 20, vorzugsweise von 10 bis 20, gemessen in Tetrahydrofuran (1%ige Lösung, 21 °C).

Vorzugsweise bestehen die Oligo(meth)acrylate zu mindestens 40 Gew.-%, besonders bevorzugt zu mindestens 60 Gew.-%, ganz besonders bevorzugt zu mindestens 80 Gew.-% aus sogenannten Hauptmonomeren. Die Hauptmonomeren sind ausgewählt aus C1-C20-Alkyl(meth)-acrylaten, Vinylestern von bis zu 20 C-Atome enthaltenden Carbonsäuren, Vinylaromaten mit bis zu 20 C-Atomen, ethylenisch ungesättigten Nitrilen, Vinylhalogeniden, Vinylethern von 1 bis 10 C-Atome enthaltenden Alkoholen, aliphatischen Kohlenwasserstoffen mit 2 bis 8 C-Atomen und 1 oder 2 Doppelbindungen oder Mischungen dieser Monomeren. Zu nennen sind z.B. (Meth)acrylsäurealkylester mit einem C1-C10-Alkylrest, wie Methylmethacrylat, Methylacrylat, n-Butylacrylat, Ethylacrylat und 2-Ethylhexylacrylat. Insbesondere sind auch Mischungen der (Meth)acrylsäurealkylester geeignet. Vinylester von Carbonsäuren mit 1 bis 20 C-Atomen sind z. B. Vinyllaurat, Vinylstearat, Vinylpropionat, Versaticsäurevinylester und Vinylacetat. Als vinylaromatische Verbindungen kommen Vinyltoluol, alpha- und p-Methylstyrol, alpha-Butylstyrol, 4-n-Butylstyrol, 4-n-Decylstyrol und vorzugsweise Styrol in Betracht. Beispiele für Nitrile sind Acrylnitril und Methacrylnitril. Die Vinylhalogenide sind mit Chlor, Fluor oder Brom substituierte ethylenisch ungesättigte Verbindungen, bevorzugt Vinylchlorid und Vinylidenchlorid. Als Vinylether zu nennen sind z. B. Vinylmethylether oder Vinylisobutylether. Bevorzugt wird Vinylether von 1 bis 4 C-Atome enthaltenden Alkoholen. Als Kohlenwasserstoffe mit 2 bis 8 C-Atomen und ein oder zwei olefinischen Doppelbindungen seien Butadien, Isopren und Chloropren, Ethylen oder Propylen genannt. Als Hauptmonomere bevorzugt sind die C1- bis C10-Alkyl(meth)-acrylate, insbesondere C1- bis C8-Alkylacrylate und -methacrylate, wobei die Acrylate jeweils besonders bevorzugt sind. Ganz besonders bevorzugt sind Methylacrylat, Ethylacrylat, n-Butylacrylat, n-Hexylacrylat, Octylacrylat und 2-Etyhlhexylacrylat sowie Mischungen dieser Monomere.

Neben den Hauptmonomeren können die Oligo(meth)acrylate weitere Monomere enthalten, z.B. Monomere mit Carbonsäure, Sulfonsäure oder Phosphonsäuregruppen. Bevorzugt sind Carbonsäuregruppen. Genannt seien z. B. Acrylsäure, Methacrylsäure, Itaconsäure, Maleinsäure oder Fumarsäure. Weitere Monomere sind z. B. auch Hydroxylgruppen enthaltende Monomere, insbesondere C1-C10-Hydroxyalkyl(meth)acrylate, (Meth)acrylamid und Ureidogruppen enthaltende Monomere wie Ureido(meth)acrylate. Als weitere Monomere seien darüber hinaus Phenyloxyethylglykolmono(meth-)acrylat, Glycidylacrylat, Glycidylmethacrylat, Amino(meth)-acrylate wie 2-Aminoethyl(meth)acrylat genannt. In Betracht kommen insbesondere auch cyclische Lactame wie N-Vinylpyrrolidon oder N-Vinylcaprolactam.

Die Oligo(meth)acrylate sind bevorzugt zu mindestens 40 Gew.-%, besonders bevorzugt zu mindestens 60 Gew.-% und ganz besonders bevorzugt zu mindestens 80 Gew.-% aus C1- bis C20-Alkyl(meth)acrylaten, insbesondere den oben genannten Alkyl(meth)acrylaten aufgebaut.

Wesentliches Merkmal des Oligo(meth)acrylats ist, dass es einen Gehalt an vernetzbaren Gruppen mit nicht-acrylischen, vernetzbaren C-C-Doppelbindungen (kurz vernetzbare Gruppen) hat. Vernetzbare Doppelbindungen sind insbesondere solche, die mit anderen Doppelbindungen radikalisch polymerisierbar sind (also durch radikalische Polymerisation vernetzen) oder solche, welche durch Abspaltung eines Wasserstoffatoms Radikale bilden (also durch Reaktionen dieser Radikale vernetzen). Als vernetzbare Gruppen in Betracht kommen z. B. die Allylgruppe oder cyclische Kohlenwasserstoffgruppen mit mindestens einer nicht-aromatischen C-C-Doppelbindung. Bei der cyclischen Kohlenwasserstoffgruppe handelt es sich insbesondere um eine Dihydrodicyclopentadienylgruppe der Formel:

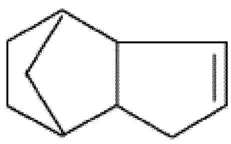

I

Die vernetzbare Gruppe kann insbesondere durch Copolymerisation mit Monomeren, welche die vernetzbare Gruppe enthalten, an das Polymer gebunden werden (vernetzbare Monomere). Geeignete vernetzbare Monomere sind z. B. Monomere, welche eine für die Polymerisation notwendige reaktive ethylenisch ungesättigte Gruppe und die vorstehende vernetzbare Gruppe enthalten. Bei der Polymerisation bleiben die vernetzbaren Gruppen zumindest zum Teil erhalten, da unter den Bedingungen der Polymerisation zunächst die reaktivere ethylenisch ungesättigte Gruppe (z. B. eine Acryl- oder Methacrylgruppe) polymerisiert. Als Monomere genannt seien Allyl(meth)acrylat oder Monomere mit einer (Meth)acrylgruppe und einer Dihydrodicyclopentadienylgruppe. Die (Meth)acrylgruppe kann direkt oder indirekt (d.h. über eine organische Gruppe als Abstandshalter bzw. Spacer) an die Dihydrodicyclopentadienylgruppe gebunden sein, bevorzugt ist Dihydrodicyclopentadienyl(meth)acrylat der Formeln:

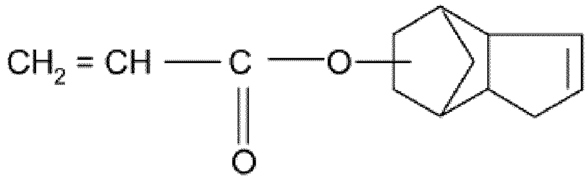

II

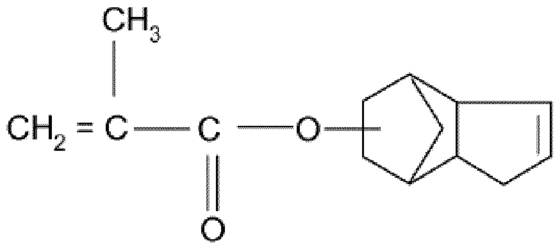

III

Das Oligo(meth)acrylat hat vorzugsweise einen Gehalt an vernetzbaren Gruppen von 0,0001 bis 0,5 Mol / 100 g Oligomer, oder von 0,0002 bis 0,1 oder von 0,001 bis 0,02 oder von 0,003 bis 0,01 Mol /100g Oligomer, besonders bevorzugt von 0,005 bis 0,25 Mol /100g Oligomer. Vorzugsweise handelt es sich um ein durch Bestrahlung mit energiereichem Licht, z. B. UV-Licht oder Elektronenstrahlen vernetzbares Oligomer. Entsprechend vernetzbar ist das Oligomer z.B. durch obige vernetzbare Gruppen oder auch, wenn Wasserstoffatome von der Polymerhauptkette photochemisch, insbesondere auch unter Verwendung eines Photoinitiators oder durch Elektronenstrahlen abgetrennt werden können, sodass ein Radikal entsteht, welches weitere chemische Reaktionen eingehen kann. Das Oligomer kann zusätzlich einen der oben beschriebenen Fotoinitiatoren in einpolymerisierter Form enthalten.

Das Oligo(meth)acrylat hat vorzugsweise eine Nullviskosität bei 23°C von kleiner als 5000 Pa s, vorzugsweise kleiner als 3000 Pa s, besonders bevorzugt kleiner als 1000 Pa s.

Die Poly(meth)acrylate und die Oligo(meth)acrylate können durch Copolymerisation der monomeren Komponenten, optional einschließlich des copolymerisierbaren Fotoinitiators unter Verwendung der üblichen Polymerisationsinitiatoren sowie optional von Reglern hergestellt werden, wobei man bei den üblichen Temperaturen in Substanz, in Emulsion, z.B. in Wasser oder flüssigen Kohlenwasserstoffen, oder in Lösung polymerisiert. Die Herstellung der Oligo(meth)-acrylate erfolgt dabei so, dass durch geeignete, das Molekulargewicht beschränkende Maßnahmen sichergestellt ist, dass deren K-Wert kleiner oder gleich 20 beträgt.

Die niedrigen Molmassen werden z.B. durch den Einsatz von Molekulargewichtsreglern oder durch den Einsatz von das Molekulargewicht regelnden Lösungsmitteln wie Isopropanol oder o-Xylol besonders gefördert. Auch Polymerisationen bei Temperaturen größer 100°C und/oder bei geringen Feststoffgehalten sind geeignet. Alternativ können Oligo(meth)acrylate auch durch Hochtemperaturmassepolymerisation unter Druck erhalten werden, wie sie in WO 03/066704 beschrieben ist.

Vorzugsweise werden die Poly(meth)acrylate und die Oligo(meth)acrylate entweder durch Emulsionspolymerisation in Wasser oder durch Polymerisation der Monomeren in organischen Lösungsmitteln, insbesondere in organischen Lösungsmitteln eines Siedebereichs von 50 bis 150 °C, vorzugsweise von 60 bis 120 °C unter Verwendung der üblichen Mengen an Polymerisationsinitiatoren, die im allgemeinen bei 0,01 bis 10, insbesondere bei 0,1 bis 4 Gew.-%, bezogen auf das Gesamtgewicht der Monomeren liegt, hergestellt. Die Polymerisate können bei Temperaturen von 20 bis 150 °C, vorzugsweise bei Temperaturen im Bereich von 70 bis 120 °C und Drucken von 0,1 bis 100 bar (absolut), bevorzugt bei 0,3 bis 10 bar, in Gegenwart von 0,01 bis 10 Gew.-% an Peroxiden oder Azostartern als Polymerisationsinitiatoren, bezogen auf die Monomeren und in Gegenwart von 0 bis 200 Gew.-% an indifferenten Lösungsmitteln, bevorzugt 5 bis 25 Gew.-%, bezogen auf die Monomeren, d.h. durch Lösungs- oder Substanzpolymerisation hergestellt werden. Vorzugsweise erfolgt die Reaktion unter zunehmendem Vakuum, z.B. durch Absenkung des Drucks von Normaldruck (1 bar) auf 500 mbar (absolut). Lösungsmittel sind beispielsweise Kohlenwasserstoffe, Alkohole wie Methanol, Ethanol, Propanol, Butanol, Isobutanol, Ketone wie Aceton, Methylethylketon, Methylisobutylketon, Essigsäureethylester, Nitrile wie Acetonitril und Benzonitril oder Gemische aus den genannten Lösemitteln. In einer bevorzugten Ausführungsform werden als Lösungsmittel für die Polymerisation ein oder mehrere Ketone mit einem Siedepunkt von unter 150°C bei Normaldruck (1 bar).

Als Polymerisationsinitiatoren kommen beispielsweise Azoverbindungen, Ketonperoxide und Alkylperoxide in Betracht, z.B. Acylperoxide wie Benzoylperoxid, Dilauroylperoxid, Didecanoylperoxid, Isononanoylperoxid, Alkylester wie tert.-Butyl-perpivalat, tert.-Butyl-per-2-ethylhexanoat, tert.-Butyl-per-maleinat, tert.-Butyl-per-isononanoat, tert.-Butyl-per-benzoat, tert.-Amylper-2-ethylhexanoat, Dialkylperoxide wie Dicumylperoxid, tert.-Butylcumylperoxid, Di-tert.-Butylperoxid und Peroxodicarbonate. Des Weiteren können als Initiatoren Azostarter wie beispielsweise 2,2'-Azobisisobutyronitril, 2,2'-Azobis(methylisobutyrat) oder 2,2'-Azobis(2,4-dimethylvaleronitril) Verwendung finden.

Für die Durchführung der Polymerisation, insbesondere zur Herstellung der Oligomere, können dem Reaktionsgemisch auch den Polymerisationsgrad senkende Verbindungen, sogenannte Polymerisationsregler zugesetzt werden, z.B. in Mengen von 0,1 bis 0,8 Gew.-Teile, bezogen auf 100 Gew.-Teile der zu polymerisierenden Monomeren. Geeignet sind z.B. Verbindungen mit einer Thiolgruppe beispielsweise Mercaptane wie Mercaptoethanol, tert.-Butylmercaptan, Mercaptobernsteinsäure, Thioglycolsäureethylhexylester, 3-Mercaptopropyltrimethoxysilan oder Dodecylmercaptan.

Nach der Polymerisation in Lösung können die Lösungsmittel gegebenenfalls unter vermindertem Druck abgetrennt werden, wobei man bei erhöhten Temperaturen, beispielsweise im Bereich von 100 bis 150 °C arbeitet. Die Polymerisate können dann in lösungsmittelfreiem Zustand (Lösungsmittelgehalt vorzugsweise kleiner 2 Gew.-%, bezogen auf die Gesamtzusammensetzung), d. h. als Schmelzen, eingesetzt werden.

Der erfindungsgemäße Schmelzklebstoff weist vorzugsweise eine Nullviskosität bei 130°C kleiner als 100 Pa s auf. Er wird in lösemittelfreier, schmelzbarer Form eingesetzt. Herstellungsbedingtes Lösungsmittel kann vorher nach geeigneten Verfahren entfernt werden, vorzugsweise auf einen Restgehalt von kleiner 0,5 Gew.%, bezogen auf den Feststoffgehalt.

Der Schmelzklebstoff kann die üblichen Zusätze enthalten wie z.B. Harze, Weichmacher, Antioxidantien, Vernetzer, usw.

Zur Herstellung der Beschichtungen werden die Schmelzhaftklebstoffe als Schmelze auf die zu beschichtenden Materialien, z.B. Substrate für Klebebänder oder Etiketten aufgetragen, wobei die Oberfläche zumindest teilweise mit einem erfindungsgemäßen Klebstoff beschichtet wird. Der Schmelzhaftklebstoff kann als Schmelze, d.h. im Allgemeinen bei Temperaturen von 50 bis 160 °C, vorzugsweise 80 bis 150 °C oder größer 100°C aufgetragen werden. Die Auftragsmenge des Haftklebstoffs ist vorzugsweise von 10 bis 100 $g/m^2$, besonders bevorzugt von 20 bis 70 $g/m^2$. Schichtdicken sind z.B. 2 bis 100 Mikrometer, vorzugsweise 10 bis 80 oder 20 bis 70 Mikrometer.

Als Träger in Betracht kommen Papier oder Polymerfilme, z.B. aus Polyester, Polyolefinen, insbesondere Polyethylen oder Polypropylen, PVC, Cellulose oder Polyacetat.

Der erfindungsgemäße strahlungsvernetzbare Schmelzklebstoff wird nach Aufbringen auf die Träger mit energiereicher Strahlung, vorzugsweise UV-Licht, insbesondere UV-C Strahlung (200-280 nm) bestrahlt, so dass eine Vernetzung erfolgt. Im Allgemeinen werden die beschichteten Substrate dazu auf ein Transportband gelegt und das Transportband an einer Strahlungsquelle, z.B. einer UV-Lampe vorbeigeführt. Der Vernetzungsgrad der Polymerisate hängt von der Dauer und Intensität der Bestrahlung ab. Vorzugsweise beträgt die Strahlungsenergie insgesamt 100 bis 1500 $mJ/cm^2$ bestrahlte Fläche. Als UV-Strahler können die üblichen Strahler, beispielsweise Quecksilbermitteldrucklampen mit einer Strahlungsleistung von 80 bis 240 Watt/cm eingesetzt werden.

Der Haftklebstoff kann zur Herstellung von Haftklebeetiketten beispielsweise auch im Transferauftrag auf Träger wie Papier oder Polymerfolien aufgebracht werden, indem er zunächst auf abhäsiv beschichteten Trägermaterialien, beispielsweise silikonisiertem Papier aufgebracht und bestrahlt wird und anschließend beispielsweise auf Papier kaschiert wird. Nach dem Abziehen des silikonisierten Papiers kann die haftklebrige Schicht optional nochmals bestrahlt werden. Die Haftklebemittel können in an sich üblicher Form modifiziert und/oder konfektioniert werden.

Auf diese Weise können Klebeartikel, insbesondere Klebeartikel mit haftklebrigen Eigenschaften hergestellt werden. Der erfindungsgemäße Schmelzklebstoff ist ein Material, welches insbesondere nach der Vernetzung durch Bestrahlung haftklebende Eigenschaften aufweist. Ein Haftklebstoff ist ein viskoelastischer Klebstoff, dessen abgebundener Film bei Raumtemperatur (20°C) in trockenem Zustand permanent klebrig und klebfähig bleibt.

Bevorzugte Klebeartikel sind Klebeetiketten, Klebebänder und selbstklebende Folien. Besonders bevorzugt sind Klebebänder. Gegenstand der Erfindung sind daher auch Klebebänder, welche auf einem bandförmigen Trägermaterial ein- oder beidseitig eine Beschichtung mit einem erfindungsgemäßen, strahlungsvernetzten Schmelzklebstoff aufweisen. Das Trägermaterial ist dabei vorzugsweise ausgewählt aus Polyethylen, Polypropylen, Cellulose, Polyacetat und Polyester.

Gegenstand der Erfindung ist auch die Verwendung des Poly(meth)acrylats zur Herstellung von Klebstoffen, vorzugsweise als Haftklebstoff zur Herstellung von Klebeetiketten, Klebebändern, Pflastern, Bandagen und selbstklebenden Folien.

Gegenstand der Erfindung sind auch Haftklebstoffartikel, wobei mindestens ein Teil einer Substratoberfläche beschichtet ist mit mindestens einem vorstehend beschriebenen Schmelzhaftklebstoff.

Die erfindungsgemäßen Klebstoffe zeichnen sich dadurch aus, dass sie bei Raumtemperatur besonders hohe Scherfestigkeiten auf Stahl aufweisen und trotzdem auf Stahl noch gute Schälfestigkeiten zeigen.

Beispiele

Einsatzstoffe:

| | |
|---|---|
| PEMA | 2-(2-Oxopyrrolidin-1-yl)ethylmethacrylat |
| PEA | 2-(2-Oxopyrrolidin-1-yl)ethylacrylat |
| Heonon-Acrylat | 2-(2-Oxooxazolidin-3-yl)ethylacrylat |
| UMA | 2-(2-Oxoimidazolidin-1-yl)ethylmethacrylat (Ureidomethacrylat) |
| MEK | Methylethylketon |

FI- Fotoinitiatormonomer:
polymerisierbarer Fotoinitiator (35%ige Lösung in MEK) der Formel F-1, wobei R1 eine C4-Kette, R2 Wasserstoff und R3 eine Phenylgruppe ist.

Beispiel 1 mit PEMA

In einer Polymerisationsapparatur bestehend aus Glasreaktor, Rückflusskühler, Rührer und Stickstoffeinlass werden in leichtem Stickstoffstrom 215 g MEK vorgelegt und auf 80 °C erwärmt. Es werden 51,1 g einer Monomerenmischung bestehend aus 745 g n-Butylacrylat, 212 g PEMA, 14,3g Photoinitiatormonomer FI (35%ig in MEK) und 50 g Acrylsäure zugegeben. Nach Wiedererreichen von 80°C werden 9,4 g einer Starterlösung aus 26,6 g tert.-Butylperpivalat (75%ig in Mineralöl) und 161,1 g MEK zugegeben und 3 min anpolymerisiert. Dann werden die restlichen 970,9 g Monomerenmischung und 178,4 g Starterlösung in 3 h zugefahren. Anschließend wird die Temperatur auf 90°C erhöht und eine Lösung von 3,2 g tert.-Butyl-perpivalat (75%ig in Mineralöl) in 24,4 g MEK in 30 min zugegeben. Danach wird Vakuum angelegt und das Lösungsmittel bei maximal 135°C und kleiner 50 mbar abdestilliert. Anschließend wird noch unter langsamen Rühren 1h bei 135°C und bestem Vakuum entgast. Die Schmelze wird in einen PP-Becher abgelassen.
K-Wert (1% in THF): 43
Nullviskosität bei 130°C: 89 Pa s,

Beispiel 2 mit PEA

In einer Polymerisationsapparatur bestehend aus Glasreaktor, Rückflusskühler, Rührer und Stickstoffeinlass werden in leichtem Stickstoffstrom 70g MEK vorgelegt und auf 80 °C erwärmt. Es werden 35,5 g einer Monomerenmischung bestehend aus 521 g n-Butylacrylat, 140g PEA, 10 g Photoinitiatormonomer FI (35%ig in MEK) und 35 g Acrylsäure zugegeben. Nach Wiedererreichen von 80°C werden 6 g einer Starterlösung aus 7,5 g tert.-Butylperpivalat (75%ig in Mineralöl) und 112,7 g MEK zugegeben und 3 min anpolymerisiert, Dann werden die restlichen 671,2 g Monomerenmischung und 114,2 g Starterlösung in 3 h zugefahren. Anschließend wird die Temperatur auf 90°C erhöht und eine Lösung von 2,24 g tert.-Butyl-perpivalat (75%ig in Mineralöl) in 17 g MEK in 30 min zugegeben. Danach wird Vakuum angelegt und das Lösungsmittel bei maximal 135°C und kleiner 50 mbar abdestilliert. Anschließend wird noch unter langsamen Rühren 1h bei 135°C und bestem Vakuum entgast. Die Schmelze wird in einen PP-Becher abgelassen.
K-Wert (1% in THF): 41
Nullviskosität bei 130°C: 55 Pa s,

Beispiel 3 mit Heonon-Acrylat

In einer Polymerisationsapparatur bestehend aus Glasreaktor, Rückflusskühler, Rührer und Stickstoffeinlass werden in leichtem Stickstoffstrom 215 g MEK vorgelegt und auf 80 °C erwärmt. Es werden 50,28 g einer Monomerenmischung bestehend aus 747 g 2-Ethylhexylacrylat, 200 g Heonon-Acrylat, 8,57 g Photoinitiatormonomer FI (35%ig in MEK) und 50 g Acrylsäure zugegeben. Nach Wiedererreichen von 80°C werden 8,39 g einer Starterlösung aus 6,67 g tert.-Butylperpivalat (75%ig in Mineralöl) und 161,1 g MEK zugegeben und 3 min anpolymerisiert. Dann werden die restlichen 955,3 g Monomerenmischung und 159,4 g Starterlösung in 3 h. zugefahren. Anschließend wird die Temperatur auf 90°C erhöht und eine Lösung von 3,2 g tert.-Butylperpivalat (75%ig in Mineralöl) in 24,4 g MEK in 30 min zugegeben. Danach wird Vakuum angelegt und das Lösungsmittel bei maximal 135°C und kleiner 50 mbar abdestilliert. Anschließend wird noch unter langsamen Rühren 1h bei 135°C und bestem Vakuum entgast. Die Schmelze wird in einen PP-Becher abgelassen.
K-Wert (1% in THF): 49,1
Nullviskosität bei 130°C: 83,3 Pa s

Beispiel 4 mit UMA (nicht anspruchsgemäß)

In einer Polymerisationsapparatur bestehend aus Glasreaktor, Rückflusskühler, Rührer und Stickstoffeinlass werden in leichtem Stickstoffstrom 371 g MEK vorgelegt und auf 80 °C erwärmt. Es werden 40,4 g einer Monomerenmischung bestehend aus 556 g 2-Ethylhexylacrylat, 200 g UMA (40%ig im 2-Ethylhexylacrylat), 11,43 g Photoinitiatormonomer FI (35%ig in MEK) und 40 g Acrylsäure zugegeben. Nach Wiedererreichen von 80°C werden 7,91 g einer Starterlösung aus 18,3 g tert.-Butylperpivalat (75%ig in Mineralöl) und 140 g MEK zugegeben und 3 min anpolymerisiert. Dann werden die restlichen 767 g Monomerenmischung und 150,2 g Starterlösung in 3 h zugefahren. Anschließend wird die Temperatur auf 90°C erhöht und eine Lösung von 2,6 g tert.-Butylperpivalat (75%ig in Mineralöl) in 19,5 g MEK in 30 min zugegeben. Danach wird Vakuum angelegt und das Lösungsmittel bei maximal 135°C und kleiner 50 mbar abdestilliert. Anschließend wird noch unter langsamen Rühren 1h bei 135°C und bestem Vakuum entgast. Die Schmelze wird in einen PP-Becher abgelassen.
K-Wert (1% in THF): 30,2
Nullviskosität bei 130°C: 33,4 Pa s

Beispiel 5: Morpholinoethylacrylat (Vergleichsbeispiel)

In einer Polymerisationsapparatur bestehend aus Glasreaktor, Rückflusskühler, Rührer und Stickstoffeinlass werden in leichtem Stickstoffstrom 200 g MEK vorgelegt und auf 80 °C erwärmt. Es werden 50,5 g einer Monomerenmischung bestehend aus 745 g n-Butylacrylat, 200 g Morpholinoethylacrylat 14,3 g Photoinitiatormonomer FI 1484 (35%ig in MEK) und 50 g Acrylsäure zugegeben. Nach Wiedererreichen von 80°C werden 2,24 g einer Starterlösung aus 1,87 g tert.-Butylperpivalat (75%ig in Mineralöl) und 42,9 g MEK zugegeben und 3 min anpolymerisiert, Dann werden die restlichen 958,8 g Monomerenmischung und 42,5 g Starterlösung in 3 h zugefahren. Anschließend wird die Temperatur auf 90°C erhöht und eine Lösung von 3,2 g tert.-Butylperpivalat (75%ig in Mineralöl) in 24,4 g MEK in 30 min zugegeben. Danach wird Vakuum angelegt und das Lösungsmittel bei maximal 135°C und kleiner 50 mbar abdestilliert. Anschließend wird noch unter langsamen Rühren 1 h bei 135°C und bestem Vakuum entgast. Die Schmelze wird in einen PP-Becher abgelassen.
K-Wert; 1% in THF: 39
Nullviskosität bei 130°C: 44 Pa s,

Messung der Nullviskosität

Die Nullviskosität ist der Grenzwert der Viskositätsfunktion bei unendlich niedrigen Scherraten. Sie wird gemessen mit einem Anton Paar Rheometer MCR 100 (US 200 Auswertesoftware) in Platte/Platte Geometrie. Die Proben werden in oszillatorischer Scherung bei kleiner Scheramplitude von 10% vermessen. Temperatur 130°C (oder wie angegeben), Kreisfrequenzrampe log 100-0,1 1/s, Messspalt 0,5 mm, Auswertung nach Carreau-Gahleitner I, Stempeldurchmesser 25 mm.

Anwendungstechnische Prüfungen:

Die Haftklebstoffe wurden mit einer Auftragsmenge von 60 $g/m^2$ auf silikonisierte PET-Folie aufgerakelt und mit UVC-Licht bestrahlt. Der Film wird dann auf eine PET-Folie Hostaphan® RN 36 als Träger übertragen. Der mit Haftklebstoff beschichtete Träger wurde in 25 mm breite Prüfstreifen geschnitten.

a) Schälfestigkeit (Adhäsion)

Zur Bestimmung der Schälfestigkeit wurden die 25 mm breiten Prüfstreifen auf die Prüffläche aus Stahl (AFERA-Stahl) bzw. Polyethylen geklebt und mit einer 1 kg schweren Rolle einmal angerollt. Der Prüfstreifen wurde dann mit einem Ende in die oberen Backen einer Zug-Dehnungs-Prüfapparatur eingespannt. Der Klebestreifen wurde mit 300 mm/min unter einem 180° Winkel von der Prüffläche abgezogen, d.h. der Klebestreifen wurde umgebogen und parallel zum Prüfblech abgezogen und der dazu benötigte Kraftaufwand gemessen. Das Maß für die Schälfestigkeit ist die Kraft in N/25 mm, die sich als Durchschnittswert aus fünf Messungen ergibt. Die Schälfestigkeit wurde 24 Stunden nach der Verklebung bestimmt. Nach dieser Zeit hat sich die Klebekraft voll ausgebildet.

b) Scherfestigkeit (Kohäsion)

Zur Bestimmung der Scherfestigkeit wurden die Prüfstreifen mit einer verklebten Fläche von 12,5 x 12,5 mm auf Stahlblech (AFERA-Stahl) geklebt, mit einer 1 kg schweren Rolle einmal angerollt und anschließend hängend mit einem 1 kg Gewicht belastet. Die Scherfestigkeit (Kohäsion) wurde bei Normklima (23°C; 50% rel. Luftfeuchtigkeit) und bei 70°C bestimmt. Das Maß für die Scherfestigkeit ist die Zeit in Stunden bis zum Abfallen des Gewichts. Es wurde jeweils der Durchschnitt aus fünf Messungen gebildet.

c) S.A.F.T.-Test (Wärmestandfestigkeit)

Die Prüfstreifen wurden mit einer verklebten Fläche von 25 x 25 mm auf AFERA-Stahl geklebt, mit einer 2 kg schweren Rolle 4 mal angerollt und nach mindestens 16 Stunden Kontaktzeit hängend mit einem 1 kg Gewicht belastet. Während der Belastung wurde ausgehend von 23°C kontinuierlich mit einer Rate von 0,5°C / min aufgeheizt. Die beim Abfallen des Gewichtes erreichte Aufheiztemperatur ist ein Maß für die Wärmestandfestigkeit des Klebstoffs. Es wurde jeweils der Durchschnitt aus drei Messungen berechnet.

Die Ergebnisse sind in Tabelle 1 zusammengefasst.

Tabelle 1: Ergebnisse der anwendungstechnischen Prüfungen

| Bsp. | Dosis1) [mJ/cm$^2$] | Schälfestigkeit [N/25 mm] | | Scherfestigkeit Stahl [h] | S.A.F.T.-Test [°C] |
|---|---|---|---|---|---|
| | | 24 h Stahl | 24 h PE | | |
| 1 | 10 | 31,6 | 4 | 16 | |
| 1 | 25 | 32,2 | 9 | 443 | 94 |
| 1 | 65 | 20,8 | 9,7 | 512 | 100 |
| 3 | 25 | 26,8 | 5,5 | 255 | |
| 3 | 65 | 25,1 | 6,0 | 202 | |
| 5 Vergleich | 65 | 22,5 | 7,5 | 41 | |

1) UV-C Strahlungsdosis

Die Ergebnisse zeigen, dass die Scherfestigkeit auf kleiner Fläche gemessen, einen hohen Wert zeigt und die Schälfestigkeit auf Stahl auch bei hoher Bestrahlungsdosis über 20 N/25 mm liegt.

## Patentansprüche

1. Schmelzklebstoff, enthaltend mindestens ein Poly(meth)acrylat, welches gebildet ist aus

   (a) mindestens einem Monomer M1, ausgewählt aus C1-bis C18-Alkyl-(meth)acrylaten, und
   (b) mindestens einem Monomer M2 der allgemeinen Formel

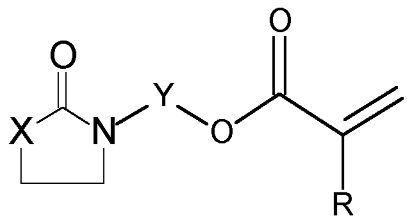

   wobei X für 0 oder $CH_2$ steht,
   Y für eine divalente organische Verbindungsgruppe, vorzugsweise eine substituierte oder nicht substituierte, lineare oder verzweigte Kette mit 2 bis 12 C-Atomen steht, die durch O, N, oder S-Atome unterbrochen sein kann, und
   R für H oder Methyl steht,

   und optional weiteren, von den Monomeren M1 und M2 verschiedenen Monomeren.

2. Schmelzklebstoff nach dem vorhergehenden Anspruch, **dadurch gekennzeichnet, dass** das Poly(meth)acrylat gebildet ist aus

   (a) zu mindestens 50 Gew.% bezogen auf die Summe aller Monomere, aus den Monomeren M1, und
   (b) zu mindestens 0,1 Gew.% bezogen auf die Summe aller Monomere, aus den Monomeren M2,
   und wobei das Poly(meth)acrylat eine Glasübergangstemperatur von kleiner oder gleich 10° C, vorzugsweise

von -60 bis +10° C aufweist; und die Glasübergangstemperatur durch Differential Scanning Calorimetrie (ASTM D 3418-08, sogenannte "midpoint temperature") bestimmt wird bei Auswertung der zweiten Heizkurve mit einer Heizrate von 20° C/min.

**3.** Schmelzklebstoff nach einem der vorhergehenden Ansprüche, **dadurch gekennzeichnet, dass** Y für eine lineare Alkylengruppe mit 2 bis 6 C-Atomen steht.

**4.** Schmelzklebstoff nach einem der vorhergehenden Ansprüche, **dadurch gekennzeichnet, dass** das Monomer M2 ausgewählt ist aus 2-(2-Oxooxazolidin-3-yl)ethylacrylat, 2-(2-Oxopyrrolidin-1-yl)ethylacrylat, 2-(2-Oxooxazolidin-3-yl)ethylmethacrylat und 2-(2-Oxopyrrolidin-l-yl)ethylmethacrylat oder Mischungen dieser Monomere.

**5.** Schmelzklebstoff nach einem der vorhergehenden Ansprüche, **dadurch gekennzeichnet, dass** der Schmelzklebstoff strahlungsvernetzbar ist, wobei der Schmelzklebstoff mindestens einen Fotoinitiator enthält und der Fotoinitiator als nicht an das Poly(meth)acrylat gebundenes Additiv vorliegt und/oder der Fotoinitiator in das Poly(meth)acrylat einpolymerisiert ist, wobei das strahlungsvernetzbare Poly(meth)acrylat vor Vernetzung eine Glasübergangstemperatur von kleiner oder gleich 10° C, vorzugsweise von -60 bis +10° C aufweist.

**6.** Schmelzklebstoff nach einem der vorhergehenden Ansprüche, **dadurch gekennzeichnet, dass** das Poly(meth)acrylat einen K-Wert von mindestens 20, vorzugsweise von 30 bis 80 aufweist; und der K-Wert gemessen wird in einer 1%igen Lösung in Tetrahydrofuran bei 21°C.

**7.** Schmelzklebstoff nach einem der vorhergehenden Ansprüche, **dadurch gekennzeichnet, dass** das Poly(meth)acrylat zu mindestens 50 Gew.% aus C4- bis C10-Alkyl(meth)acrylaten, vorzugsweise ausgewählt aus n-Butylacrylat und 2-Ethylhexylacrylat gebildet ist.

**8.** Schmelzklebstoff nach einem der Ansprüche 5 bis 7, **dadurch gekennzeichnet, dass** das strahlungsvernetzbare Poly(meth)acrylat durch Bestrahlung mit UV-Licht vernetzbar ist.

**9.** Schmelzklebstoff nach einem der Ansprüche 5 bis 8, **dadurch gekennzeichnet, dass** der Fotoinitiator in Form eines ethylenisch ungesättigten, copolymerisierbaren Fotoinitiators in einer Menge von 0,05 bis 5 Gew.% in das Poly(meth)acrylat copolymerisiert ist.

**10.** Schmelzklebstoff nach einem der Ansprüche 5 bis 9, **dadurch gekennzeichnet, dass** der Fotoinitiator in nicht einpolymerisierter Form die allgemeine Struktur

A-X-B

aufweist, wobei

A ein einwertiger organischer Rest ist, welcher eine Phenongruppe aufweist,
X eine Estergruppe ist, ausgewählt aus -O-C(=O)-, -(C=O)-O- und -O-(C=O)-O-,
und B ein einwertiger organischer Rest ist, welcher eine ethylenisch ungesättigte, radikalisch polymerisierbare Gruppe enthält.

**11.** Schmelzklebstoff nach dem vorhergehenden Anspruch, **dadurch gekennzeichnet, dass** der Fotoinitiator in nicht einpolymerisierter Form die allgemeine Struktur

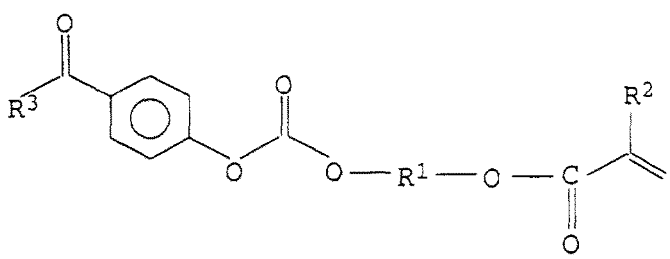

aufweist, worin R1 für einen divalenten organischen Rest mit bis zu 30 C-Atomen, R2 für ein H-Atom oder eine

Methylgruppe und R3 für eine substituierte oder unsubstituierte Phenylgruppe oder für eine C1-C4-Alkylgruppe steht.

12. Schmelzklebstoff nach einem der vorhergehenden Ansprüche, **dadurch gekennzeichnet, dass** der Schmelzklebstoff mindestens ein Poly(meth)acrylat enthält, wobei das Poly(meth)acrylat zusätzlich zu den Monomeren M1 und M2 aus mindestens einem Monomeren mit polaren Gruppen gebildet ist, wobei die polaren Gruppen ausgewählt sind aus Carbonsäuregruppen, Carbonsäureanhydridgruppen, Hydroxygruppen, Amidgruppen, Urethangruppen, Harnstoffgruppen, Piperidinylgruppen, Piperazinylgruppen, Morpholinylgruppen, Imidazolylgruppen und Kombinationen von zwei oder mehr der genannten Gruppen.

13. Schmelzklebstoff nach dem vorhergehenden Anspruch, **dadurch gekennzeichnet, dass** das Poly(meth)acrylatpolymer zu 0,1 bis 30 Gew.%, bevorzugt zu 0,3 bis 25 Gew.% aus den Monomeren mit polaren Gruppen gebildet ist.

14. Schmelzklebstoff nach einem der vorhergehenden Ansprüche, **dadurch gekennzeichnet, dass** der Schmelzklebstoff mindestens ein Poly(meth)acrylat enthält, wobei das Poly(meth)acrylat gebildet ist aus

(a1) mindestens 60 Gew.% mindestens eines Monomer M1, welches ausgewählt ist aus der Gruppe bestehend aus n-Butylacrylat, n-Hexylacrylat, 2-Ethyl-hexylacrylat, Propylheptylacrylat und deren Mischungen und
(a2) 0,5 bis 15 Gew.% Monomeren mit polaren Gruppen, wobei die polaren Gruppen ausgewählt sind aus Carbonsäuregruppen,
(b) 0,2 bis 25 Gew.% Monomeren M2, ausgewählt aus 2-(2-Oxooxazolidin-3-yl)ethyl(meth)acrylat und 2-(2-Oxopyrrolidin-1-yl)ethyl(meth)acrylat und Mischungen dieser Monomere und
(c) 0,05 bis 5 Gew.% mindestens eines ethylenisch ungesättigten, copolymerisierbaren Fotoinitiators.

15. Poly(meth)acrylat, geeignet für einen Schmelzklebstoff, wobei das Poly(meth)acrylat, gebildet ist aus

(a) mindestens einem Monomer M1, ausgewählt aus C1-bis C18-Alkyl-(meth)acrylaten, und
(b) mindestens einem Monomer M2 der allgemeinen Formel

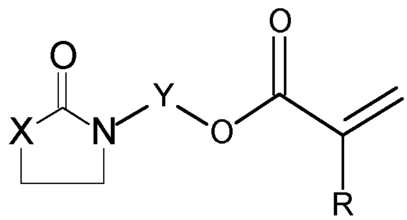

wobei X für 0 oder $CH_2$ steht,
Y für eine divalente organische Verbindungsgruppe, vorzugsweise eine substituierte oder nicht substituierte, lineare oder verzweigte Kette mit 2 bis 12 C-Atomen steht, die durch 0, N, oder S-Atome unterbrochen sein kann, und
R für H oder Methyl steht,

und mindestens einem in das Poly(meth)acrylat einpolymerisierten Fotoinitiator.

16. Poly(meth)acrylat nach dem vorhergehenden Anspruch, **dadurch gekennzeichnet, dass** das Poly(meth)acrylat gebildet ist aus

(a1) mindestens 60 Gew.% mindestens eines Monomer M1, welches ausgewählt ist aus der Gruppe bestehend aus n-Butylacrylat, n-Hexylacrylat, 2-Ethyl-hexylacrylat, Propylheptylacrylat und deren Mischungen und
(a2) 0,5 bis 15 Gew.% Monomeren mit polaren Gruppen, wobei die polaren Gruppen ausgewählt sind aus Carbonsäuregruppen,
(b) 0,2 bis 25 Gew.% Monomeren M2, ausgewählt aus 2-(2-Oxooxazolidin-3-yl)ethyl(meth)acrylat und 2-(2-Oxopyrrolidin-1-yl)ethyl(meth)acrylat und Mischungen dieser Monomere und
(c) 0,05 bis 5 Gew.% mindestens eines ethylenisch ungesättigten, copolymerisierbaren Fotoinitiators.

17. Verwendung des Poly(meth)acrylats nach einem der beiden vorhergehenden Ansprüche zur Herstellung von Klebstoffen, vorzugsweise als Haftklebstoff zur Herstellung von Klebeetiketten, Klebebändern, Pflastern, Bandagen und selbstklebenden Folien.

18. Haftklebstoffartikel, wobei mindestens ein Teil einer Substratoberfläche beschichtet ist mit mindestens einem Schmelzklebstoff gemäß einem der Ansprüche 1 bis 14.

## Claims

1. A hotmelt adhesive comprising at least one poly(meth)acrylate which is formed from

(a) at least one monomer M1 selected from C1 to C18 alkyl (meth)acrylates, and
(b) at least one monomer M2 of the general formula

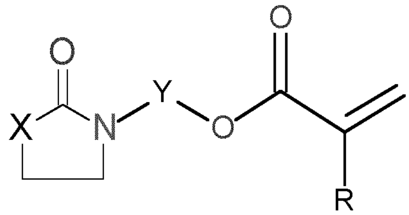

where X is O or $CH_2$,
Y is a divalent organic connecting group, preferably a substituted or unsubstituted, linear or branched chain having 2 to 12 C atoms, which may be interrupted by O, N, or S atoms, and
R is H or methyl,

and optionally further monomers different from the monomers M1 and M2.

2. The hotmelt adhesive according to the preceding claim, wherein the poly(meth)acrylate is formed from

(a) the monomers M1 to an extent of at least 50 wt%, based on the sum of all the monomers, and
(b) the monomers M2 to an extent of at least 0.1 wt%, based on the sum of all the monomers,
and where the poly(meth)acrylate has a glass transition temperature of less than or equal to 10°C, preferably of -60 to +10°C, the glass transition temperature being determined by differential scanning calorimetry (ASTM D 3418-08, midpoint temperature) on evaluation of the second heating curve at a heating rate of 20°C/min.

3. The hotmelt adhesive according to either of the preceding claims, wherein Y is a linear alkylene group having 2 to 6 C atoms.

4. The hotmelt adhesive according to any of the preceding claims, wherein the monomer M2 is selected from 2-(2-oxooxazolidin-3-yl)ethyl acrylate, 2-(2-oxopyrrolidin-1-yl)ethyl acrylate, 2-(2-oxooxazolidin-3-yl)ethyl methacrylate, and 2-(2-oxopyrrolidin-1-yl)ethyl methacrylate, or mixtures of these monomers.

5. The hotmelt adhesive according to any of the preceding claims, wherein the hotmelt adhesive is radiation-crosslinkable, the hotmelt adhesive comprising at least one photoinitiator and the photoinitiator being present as an additive not bonded to the poly(meth)acrylate, and/or the photoinitiator having been incorporated into the poly(meth)acrylate by copolymerization, the radiation-crosslinkable poly(meth)acrylate having a glass transition temperature before crosslinking of less than or equal to 10°C, preferably of -60 to +10°C.

6. The hotmelt adhesive according to any of the preceding claims, wherein the poly(meth)acrylate has a K value of at least 20, preferably of 30 to 80, the K value being measured in a 1% strength solution in tetrahydrofuran at 21°C.

7. The hotmelt adhesive according to any of the preceding claims, wherein the poly(meth)acrylate is formed to an extent of at least 50 wt% of C4 to C10 alkyl (meth)acrylates, preferably selected from n-butyl acrylate and 2-ethylhexyl acrylate.

8. The hotmelt adhesive according to any of claims 5 to 7, wherein the radiation-crosslinkable poly(meth)acrylate is crosslinkable by irradiation with UV light.

9. The hotmelt adhesive according to any of claims 5 to 8, wherein the photoinitiator has been copolymerized into the

poly(meth)acrylate in the form of an ethylenically unsaturated, copolymerizable photoinitiator in an amount of 0.05 to 5 wt%.

**10.** The hotmelt adhesive according to any of claims 5 to 9, wherein the photoinitiator in uncopolymerized form has the general structure

A-X-B,

where

A is a monovalent organic radical which has a phenone group,
X is an ester group selected from -O-C(=O)-, -(C=O)-O, and -O-(C=O)-O-,
and B is a monovalent organic radical which comprises an ethylenically unsaturated, radically polymerizable group.

**11.** The hotmelt adhesive according to the preceding claim, wherein the photoinitiator in uncopolymerized form has the general structure

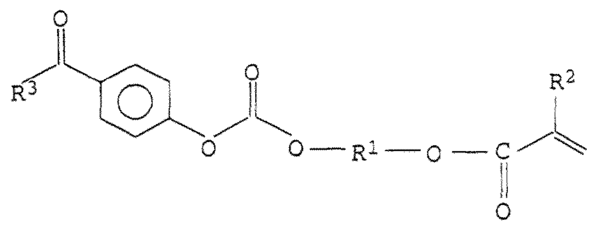

in which R1 is a divalent organic radical having up to 30 C atoms, R2 is an H atom or a methyl group, and R3 is a substituted or unsubstituted phenyl group or is a C1-C4 alkyl group.

**12.** The hotmelt adhesive according to any of the preceding claims, wherein the hotmelt adhesive comprises at least one poly(meth)acrylate, the poly(meth)acrylate being formed, additionally to the monomers M1 and M2, from at least one monomer having polar groups, the polar groups being selected from carboxylic acid groups, carboxylic anhydride groups, hydroxyl groups, amide groups, urethane groups, urea groups, piperidyl groups, piperazinyl groups, morpholinyl groups, imidazolyl groups, and combinations of two or more of the stated groups.

**13.** The hotmelt adhesive according to the preceding claim, wherein the poly(meth)acrylate polymer is formed to an extent of 0.1 to 30 wt%, preferably of 0.3 to 25 wt%, of the monomers having polar groups.

**14.** The hotmelt adhesive according to any of the preceding claims, wherein the hotmelt adhesive comprises at least one poly(meth)acrylate, the poly(meth)acrylate being formed from

(a1) at least 60 wt% of at least one monomer M1 which is selected from the group consisting of n-butyl acrylate, n-hexyl acrylate, 2-ethylhexyl acrylate, propylheptyl acrylate, and mixtures thereof, and
(a2) 0.5 to 15 wt% of monomers having polar groups, the polar groups being selected from carboxylic acid groups,
(b) 0.2 to 25 wt% of monomers M2, selected from 2-(2-oxooxazolidin-3-yl)ethyl (meth)acrylate and 2-(2-oxopyrrolidin-1-yl)ethyl (meth)acrylate, and mixtures of these monomers, and
(c) 0.05 to 5 wt% of at least one ethylenically unsaturated, copolymerizable photoinitiator.

**15.** A poly(meth)acrylate suitable for a hotmelt adhesive, the poly(meth)acrylate being formed from

(a) at least one monomer M1 selected from C1 to C18 alkyl (meth)acrylates, and
(b) at least one monomer M2 of the general formula

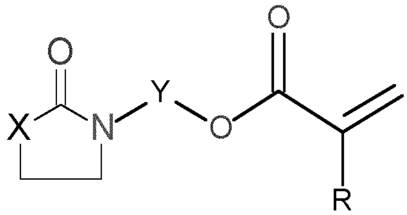

where X is O or $CH_2$,
Y is a divalent organic connecting group, preferably a substituted or unsubstituted, linear or branched chain having 2 to 12 C atoms, which may be interrupted by O, N, or S atoms, and
R is H or methyl,

and at least one photoinitiator copolymerized into the poly(meth)acrylate.

**16.** The poly(meth)acrylate according to the preceding claim, wherein the poly(meth)acrylate is formed from

(a1) at least 60 wt% of at least one monomer M1 which is selected from the group consisting of n-butyl acrylate, n-hexyl acrylate, 2-ethylhexyl acrylate, propylheptyl acrylate, and mixtures thereof, and
(a2) 0.5 to 15 wt% of monomers having polar groups, the polar groups being selected from carboxylic acid groups,
(b) 0.2 to 25 wt% of monomers M2, selected from 2-(2-oxooxazolidin-3-yl)ethyl (meth)acrylate and 2-(2-oxopyrrolidin-1-yl)ethyl (meth)acrylate, and mixtures of these monomers, and
(c) 0.05 to 5 wt% of at least one ethylenically unsaturated, copolymerizable photoinitiator.

**17.** The use of the poly(meth)acrylate according to either of the two preceding claims for producing adhesives, preferably as pressure-sensitive adhesive for producing adhesive labels, adhesive tapes, plasters, bandages, and self-adhesive sheets.

**18.** A pressure-sensitive adhesive article where at least part of a substrate surface has been coated with at least one hotmelt adhesive according to any of claims 1 to 14.

**Revendications**

**1.** Adhésif fusible, contenant au moins un poly(méth)acrylate, qui est formé à partir de

(a) au moins un monomère M1, choisi parmi les (méth)acrylates d'alkyle en C1 à C18, et
(b) au moins un monomère M2 de la formule générale

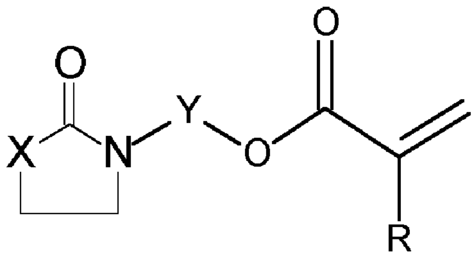

dans laquelle X représente O ou $CH_2$,
Y représente un groupe de liaison organique bivalent, de préférence une chaîne substituée ou non substituée, linéaire ou ramifiée, de 2 à 12 atomes C, qui peut être interrompue par des atomes O, N ou S, et
R représente H ou méthyle,

et éventuellement d'autres monomères, différents des monomères M1 et M2.

**2.** Adhésif fusible selon la revendication précédente, **caractérisé en ce que** le poly(méth)acrylate est formé à partir de

(a) au moins 50 % en poids, par rapport à la somme de tous les monomères, des monomères M1, et
(b) au moins 0,1 % en poids, par rapport à la somme de tous les monomères, des monomères M2,
et dans lequel le poly(méth)acrylate présente une température de transition vitreuse inférieure ou égale à 10 °C, de préférence de -60 à +10 °C ; et la température de transition vitreuse est déterminée par calorimétrie différentielle à balayage (ASTM D 3418-08, dite « midpoint temperature ») lors de l'évaluation de la deuxième courbe de chauffage avec un taux de chauffage de 20 °C/min.

3. Adhésif fusible selon l'une quelconque des revendications précédentes, **caractérisé en ce qu'**Y représente un groupe alkylène linéaire de 2 à 6 atomes C.

4. Adhésif fusible selon l'une quelconque des revendications précédentes, **caractérisé en ce que** le monomère M2 est choisi parmi l'acrylate de 2-(2-oxooxazolidin-3-yl)éthyle, l'acrylate de 2-(2-oxopyrrolidin-1-yl)éthyle, le méthacrylate de 2-(2-oxooxazolidin-3-yl)éthyle et le méthacrylate de 2-(2-oxopyrrolidin-1-yl)éthyle ou les mélanges de ces monomères.

5. Adhésif fusible selon l'une quelconque des revendications précédentes, **caractérisé en ce que** l'adhésif fusible est réticulable par rayonnement, l'adhésif fusible contenant au moins un photoinitiateur et le photoinitiateur se présentant sous la forme d'un additif non relié au poly(méth)acrylate et/ou le photoinitiateur étant polymérisé dans le poly(méth)acrylate, le poly(méth)acrylate réticulable par rayonnement présentant avant la réticulation une température de transition vitreuse inférieure ou égale à 10 °C, de préférence de -60 à +10 °C.

6. Adhésif fusible selon l'une quelconque des revendications précédentes, **caractérisé en ce que** le poly(méth)acrylate présente une valeur K d'au moins 20, de préférence de 30 à 80 ; et la valeur K est mesurée dans une solution à 1 % dans du tétrahydrofurane à 21 °C.

7. Adhésif fusible selon l'une quelconque des revendications précédentes, **caractérisé en ce que** le poly(méth)acrylate est formé à hauteur d'au moins 50 % en poids par des (méth)acrylates d'alkyle en C4 à C10, de préférence choisis parmi l'acrylate de n-butyle et l'acrylate de 2-éthylhexyle.

8. Adhésif fusible selon l'une quelconque des revendications 5 à 7, **caractérisé en ce que** le poly(méth)acrylate réticulable par rayonnement est réticulable par exposition à de la lumière UV.

9. Adhésif fusible selon l'une quelconque des revendications 5 à 8, **caractérisé en ce que** le photoinitiateur est copolymérisé dans le poly(méth)acrylate sous la forme d'un photoinitiateur éthyléniquement insaturé, copolymérisable, en une quantité de 0,05 à 5 % en poids.

10. Adhésif fusible selon l'une quelconque des revendications 5 à 9, **caractérisé en ce que** le photoinitiateur présente sous forme non polymérisée la structure générale

A-X-B,

dans laquelle

A est un radical organique monovalent, qui comprend un groupe phénone,
X est un groupe ester, choisi parmi -0-C(=0)-, -(C=O)-O- et -O-(C=O)-O-,
et B est un radical organique monovalent, qui contient un groupe éthyléniquement insaturé, polymérisable par voie radicalaire.

11. Adhésif fusible selon la revendication précédente, **caractérisé en ce que** le photoinitiateur présente sous forme non polymérisée la structure suivante

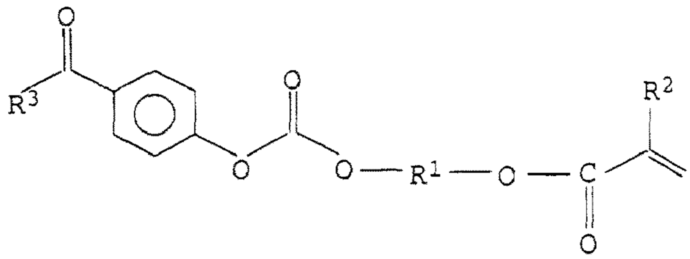

dans laquelle R1 représente un radical organique bivalent contenant jusqu'à 30 atomes C, R2 représente un atome H ou un groupe méthyle, et R3 représente un groupe phényle substitué ou non substitué ou un groupe alkyle en C1-C4.

**12.** Adhésif fusible selon l'une quelconque des revendications précédentes, **caractérisé en ce que** l'adhésif fusible contient au moins un poly(méth)acrylate, le poly(méth)acrylate étant formé en plus des monomères M1 et M2 à partir d'au moins un monomère contenant des groupes polaires, les groupes polaires étant choisis parmi les groupes acide carboxylique, les groupes anhydride d'acide carboxylique, les groupes hydroxy, les groupes amide, les groupes uréthane, les groupes urée, les groupes pipéridinyle, les groupes pipérazinyle, les groupes morpholinyle, les groupes imidazolyle et les combinaisons de deux ou plus des groupes mentionnés.

**13.** Adhésif fusible selon la revendication précédente, **caractérisé en ce que** le polymère de poly(méth)acrylate est formé à hauteur de 0,1 à 30 % en poids, de préférence à hauteur de 0,3 à 25 % en poids, à partir des monomères contenant des groupes polaires.

**14.** Adhésif fusible selon l'une quelconque des revendications précédentes, **caractérisé en ce que** l'adhésif fusible contient au moins un poly(méth)acrylate, le poly(méth)acrylate étant formé à partir de

(a1) au moins 60 % en poids d'au moins un monomère M1, qui est choisi dans le groupe constitué par l'acrylate de n-butyle, l'acrylate de n-hexyle, l'acrylate de 2-éthyl-hexyle, l'acrylate de propylheptyle et leurs mélanges, et
(a2) 0,5 à 15 % en poids de monomères contenant des groupes polaires, les groupes polaires étant choisis parmi les groupes acide carboxylique,
(b) 0,2 à 25 % en poids de monomères M2, choisis parmi le (méth)acrylate de 2-(2-oxooxazolidin-3-yl)éthyle et le (méth)acrylate de 2-(2-oxopyrrolidin-1-yl)éthyle et les mélanges de ces monomères, et
(c) 0, 05 à 5 % en poids d'au moins un photoinitiateur éthyléniquement insaturé, copolymérisable.

**15.** Poly(méth)acrylate, approprié pour un adhésif fusible, le poly(méth)acrylate étant formé à partir de

(a) au moins un monomère M1, choisi parmi les (méth)acrylates d'alkyle en C1 à C18, et
(b) au moins un monomère M2 de la formule générale

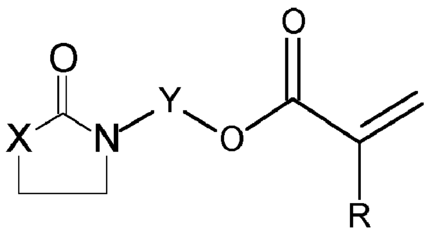

dans laquelle X représente O ou $CH_2$,
Y représente un groupe de liaison organique bivalent, de préférence une chaîne substituée ou non substituée, linéaire ou ramifiée, de 2 à 12 atomes C, qui peut être interrompue par des atomes O, N ou S, et
R représente H ou méthyle,

et au moins un photoinitiateur polymérisé dans le poly(méth)acrylate.

**16.** Poly(méth)acrylate selon la revendication précédente, **caractérisé en ce que** le poly(méth)acrylate est formé à

partir de

(a1) au moins 60 % en poids d'au moins un monomère M1, qui est choisi dans le groupe constitué par l'acrylate de n-butyle, l'acrylate de n-hexyle, l'acrylate de 2-éthyl-hexyle, l'acrylate de propylheptyle et leurs mélanges, et
(a2) 0,5 à 15 % en poids de monomères contenant des groupes polaires, les groupes polaires étant choisis parmi les groupes acide carboxylique,
(b) 0,2 à 25 % en poids de monomères M2, choisis parmi le (méth)acrylate de 2-(2-oxooxazolidin-3-yl)éthyle et le (méth)acrylate de 2-(2-oxopyrrolidin-1-yl)éthyle et les mélanges de ces monomères, et
(c) 0,05 à 5 % en poids d'au moins un photoinitiateur éthyléniquement insaturé, copolymérisable.

**17.** Utilisation du poly(méth)acrylate selon l'une quelconque des deux revendications précédentes pour la fabrication d'adhésifs, de préférence en tant qu'adhésif sensible à la pression pour la fabrication d'étiquettes adhésives, de bandes adhésives, de pansements, de bandages et de films autocollants.

**18.** Article adhésif sensible à la pression, dans lequel au moins une partie de la surface de substrat est revêtue avec au moins un adhésif fusible selon l'une quelconque des revendications 1 à 14.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 102004058070
- EP 246848 A
- EP 377191 A
- EP 445641 A
- EP 1132444 A2
- WO 0123488 A
- WO 2012139941 A
- WO 2012140174 A
- WO 2014154507 A
- EP 1213306 A
- WO 03066704 A


### In der Beschreibung aufgeführte Nicht-Patentliteratur


- **T.G. FOX.** *Bull. Am. Phys. Soc.,* 1956, 123
- Ullmann's Encyclopädie der technischen Chemie. Verlag Chemie, 1980, vol. 19, 18
- Ullmann's Ecyclopedia of Industrial Chemistry. VCH Weinheim, 1992, vol. A21, 169
- **J. BRANDRUP ; E.H. IMMERGUT.** Polymer Handbook. J. Wiley, 1966
- POLYMER HANDBOOK. J. Wiley, 1975
- POLYMER HANDBOOK. J. Wiley, 1989